(19) Europäisches Patentamt
European Patent Office
Office européen des brevets

(11) **EP 3 434 228 B1**

(12) **FASCICULE DE BREVET EUROPEEN**

(45) Date de publication et mention
de la délivrance du brevet:
**20.11.2019 Bulletin 2019/47**

(51) Int Cl.:
***A61F 2/42*** *(2006.01)*    ***A61B 17/56*** *(2006.01)*

(21) Numéro de dépôt: **18185064.5**

(22) Date de dépôt: **23.07.2018**

(54) **IMPLANT SOUS-TALIEN POUR ARTHRORISE DE L'ARTICULATION TALO-CALCANÉENNE**

SUBTALARES IMPLANTAT FÜR ARTHRORISE DES SPRUNGGELENKS

SUBTALAR IMPLANT FOR ARTHROEREISIS OF THE TALOCALCANEAN JOINT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.07.2017 FR 1757029**

(43) Date de publication de la demande:
**30.01.2019 Bulletin 2019/05**

(73) Titulaire: **Neosteo**
**44100 Nantes (FR)**

(72) Inventeurs:
• **DECHELETTE, Maxime**
**44390 Petit Mars (FR)**

• **SORIN, Sylvain**
**44100 Nantes (FR)**

(74) Mandataire: **Gicquel, Frédéric**
**Legi LC**
**4 impasse des Jades**
**CS 63818**
**44338 Nantes Cedex 3 (FR)**

(56) Documents cités:
**EP-A2- 3 061 423     WO-A1-2013/123366**
**US-A1- 2005 187 636     US-A1- 2013 304 224**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

Le domaine de l'invention est celui de la conception et de la fabrication de dispositifs médicaux implantables pour arthrorise. Plus précisément, l'invention concerne un implant extra osseux, pour arthrorise de l'articulation talo-calcanéenne, l'implant étant destiné à être inséré dans l'articulation talo-calcanéenne par son orifice latéral.

L'arthrorise sous talienne est une opération chirurgicale pratiquée dans le cadre de la cure du pied plat flexible, en particulier chez l'enfant et l'adolescent.

Le syndrome du pied plat se caractérise par une instabilité de l'articulation talo-calcanéenne. Cette instabilité provoque un phénomène d'hyper pronation lors de la mise en charge du pied, ce qui aboutit à un affaissement de l'arche plantaire. Les conséquences cliniques principales sont :

- des douleurs ;
- des troubles de la marche ;
- une rigidité du tendon d'Achille lors de la marche ;
- une détérioration des articulations périphériques due à une modification de la distribution des charges.

L'arthrorise sous-talienne permet de limiter le mouvement de l'articulation en provoquant un blocage grâce à un implant.

Pour des étapes peu avancées de pieds plats, et notamment lorsque le pied plat est dit « flexible », l'arthrorise est une intervention qui cumule plusieurs bénéfices cliniques par rapport à d'autres options thérapeutiques.

En effet, l'arthrorise constitue une intervention rapide, qui nécessite une incision minime et qui n'entraîne pas de coupe osseuse. L'arthrorise permet également de conserver une correction obtenue et ce même après l'extraction de l'implant utilisé pour la réalisation de la correction. Aussi, il s'agit d'une opération qui est réversible en cas de complications grâce à un simple retrait de l'implant tel qu'évoqué précédemment.

Pour réaliser l'arthrorise, l'implant (généralement une vis extra osseuse) est inséré dans le sinus du tarse orienté dans une position antéro-latérale vers une position postério-médiale. En d'autres termes, l'implant est inséré dans l'articulation talo-calcanéenne par son orifice latéral.

Suite à l'insertion, l'implant doit reposer sur la surface du calcanéum en « flottement libre ». Après immobilisation, des tissus fibreux, dits tissus colonisateurs, doivent coloniser des aspérités et des cavités de l'implant de manière à le maintenir en place in situ.

Selon une conception classique actuelle, les implants sous-taliens pour arthrorise de l'articulation talo-calcanéenne prennent tous la forme d'une vis, c'est-à-dire une forme oblongue généralement conique de révolution munie d'une pluralité de rainures hélicoïdales. Les rainures hélicoïdales forment un pas de vis qui n'est pas destiné à faire avancer l'implant en le vissant, mais uniquement à servir de support pour la repousse fibreuse des tissus colonisateurs. En effet, les tissus colonisateurs se fixent dans les rainures hélicoïdales pour produire une résistance en traction selon l'axe d'insertion de l'implant.

Cette conception classique des implants présente néanmoins des inconvénients.

En effet, des complications récurrentes amenant au retrait de l'implant sont relevées dans la littérature.

Une principale complication réside en un recul de l'implant, et potentiellement à la création d'une gêne liée au recul de l'implant (inflammation du sinus du tarse, synovite, ...).

Le recul de l'implant est généralement attribué à la forme de l'implant. En effet, lors de la marche, un effort s'opère sur le contour de révolution généralement conique de l'implant. Cet effort prend la forme de micromouvements et entraîne un phénomène de dévissage de l'implant.

Les efforts sur le contour de révolution généralement conique de l'implant se transforment en effet en un mouvement de translation axial, ce qui entraîne le phénomène de dévissage à cause du filet hélicoïdal (rainure hélicoïdale ou pas de vis).

Afin d'offrir un meilleur ancrage pour les tissus, plusieurs solutions ont été proposées :

- le document de brevet publié sous le n° US 8,092,547 B2 propose d'intégrer des trous sur la périphérie de l'implant, ces trous débouchant ou non sur une canule. L'implant a ainsi une forme conique présentant un filet d'ancrage sur la surface radialement extérieure du cône ;
- le document de brevet publié sous le n° US 2009/0099664 A1, propose un implant conique présentant sur sa surface radialement extérieure un filet d'ancrage, et intégrant des rainures à l'intérieur des creux présentés par le filet d'ancrage ;
- les documents de brevet publiés sous les numéros US 8,092,547 B2 et WO 2012/100054 A1 proposent d'intégrer des profils de filets dits négatifs, c'est-à-dire produisant une contre-dépouille.

Le document de brevet publié sous le n° WO 2012/100054 A1, cité précédemment, propose également un implant qui ne présente pas de partie conique et qui présente, à la place de cette partie conique, une partie sphéroïde de manière à fournir un blocage uniforme de l'articulation lors de la présence d'une particularité anatomique ou de la technique d'implantation du chirurgien. En effet, ce document précise qu'un implant conique peut aisément être implanté de telle façon qu'il produit une pression trop faible ou trop forte sur l'articulation talo-calcanéenne.

L'art antérieur propose enfin le document de brevet publié sous le n° US 9,125,701 B2 qui décrit un implant réalisé à l'aide de plusieurs composants et qui est basé sur le principe de la vis-cheville expansive. La mise en oeuvre d'un tel implant est plus compliquée et plus onéreuse qu'un implant monobloc usiné. Aussi, des complications mécaniques dues à la nature de l'assemblage peuvent aussi survenir.

Il est également connu le document de brevet publié sous le numéro US 2013/0304224 A1 qui décrit un implant comprenant un corps principal et des éléments de fixation indépendants du corps principal. Les éléments de fixation consistent en des vis à insérer dans le corps principal, ces vis étant conçues pour s'étendre hors du corps principal de manière à s'ancrer dans des structures osseuses. L'implant décrit dans ce document est conçu pour réaliser une arthrodèse, c'est-à-dire qu'il a pour but d'amener à la réalisation d'une fusion osseuse sur une articulation pathologique. Un tel implant est inadapté à la réalisation d'une arthrorise.

Les solutions proposées par l'art antérieur ont pour but d'offrir un meilleur ancrage pour les tissus colonisateurs mais peuvent néanmoins participer au phénomène d'irritation voire d'érosion osseuse. Les implants présentés par l'art antérieur induisent aussi de manière constante une propension au dévissage qui n'est pas complétement résolue par les solutions proposées.

L'invention a notamment pour objectif de résoudre les inconvénients de l'art antérieur.

L'invention a notamment pour objectif de proposer un implant sous-talien pour arthrorise de l'articulation talo-calcanéenne, extra-osseux, qui n'a pas ou à tout le moins a peu tendance à reculer une fois qu'il a été inséré et que la colonisation des tissus fibreux a eu lieu, sous l'effet de micromouvements liés à la marche.

L'invention a également pour objet de proposer un tel implant sous-talien qui n'a pas tendance à irriter les tissus et les structures osseuses environnants.

L'invention a encore pour objectif de proposer un tel implant sous-talien qui ne présente pas une mise en œuvre compliquée et onéreuse.

Ces objectifs ainsi que d'autres qui apparaitront par la suite, sont atteints par l'invention qui a pour objet un implant sous-talien pour arthrorise de l'articulation talo-calcanéenne, l'implant étant destiné à être inséré dans l'articulation talo-calcanéenne par son orifice latéral et présentant une forme oblongue qui s'étend longitudinalement selon un axe d'insertion de l'implant, l'implant étant monobloc et comportant :

- une tête (2) avec une forme de cône de révolution centrée sur l'axe d'insertion ;
- un filetage d'ancrage (4) ; et
- une tige (3) avec une forme de cylindre de révolution centrée sur l'axe d'insertion, la tige présentant à sa surface le filetage d'ancrage et étant destinée à être insérée jusqu'à l'axe de rotation de l'articulation talo-calcanéenne, la tête s'étendant en s'évasant à partir de la tige et étant située en amont de la tête selon l'axe d'insertion de l'implant, la tête présentant à sa surface radialement extérieure des moyens de retenue (5) en rotation de l'implant autour de l'axe d'insertion, les moyens de retenue en rotation étant destinés à exercer une retenue contre un tissu colonisateur,

- des dépressions (51) par rapport à la forme de cône de révolution de la tête (2) ;
- au moins un évidement (52) situé à l'intérieur des dépressions (51), le ou les évidements (52) communiquant ensemble à l'intérieur de la tête (2) pour créer au moins une cavité annulaire (6) destinée à être colonisée par du tissu colonisateur,

caractérisé en ce que, selon une coupe transversale perpendiculaire à l'axe d'insertion :

- la tête (2) s'inscrit à l'intérieur d'un polygone (P) dont les côtés s'étendent au niveau des dépressions (51) ;
- la ou les cavités annulaires présentent une forme polygonale apte à permettre l'inscription d'un polygone de fibro-intégration à l'intérieur de la tête.

Un tel implant sous-talien pour arthrorise de l'articulation talo-calcanéenne ne présente pas ou peu de propension au dévissage sous l'action de micromouvements due au cycle de marche.

En effet, grâce au positionnement du filetage d'ancrage sur la surface de la tige, et à l'insertion de la tige jusqu'à l'axe de rotation de l'articulation talo-calcanéenne, les mouvements au niveau de l'axe de l'articulation sont nuls ou à tout le moins très réduits à proximité de ce filetage d'ancrage. Ceci a pour effet que l'implant présente un risque quasi nul de dévissage intempestif lié à la présence de ce filetage d'ancrage.

Egalement, la tête de l'implant présente une forme de cône de révolution centré sur l'axe d'insertion. Cette tête permet une pénétration homogène et progressive de l'implant lors de sa mise en position selon l'axe d'insertion par l'orifice latéral de l'articulation talo-calcanéenne. La forme de la tête autorise une correction précise du syndrome du pied plat.

En complément du positionnement du filetage d'ancrage et pour limiter l'impact sur l'implant de micromouvements liés à la marche, les moyens de retenue en rotation présentés à la surface de la tête luttent contre toute rotation de l'implant autour de son axe d'insertion. Ces moyens de blocage en rotation vont ainsi coopérer avec la position du filetage d'ancrage pour éviter tout recul de l'implant suite à son implantation.

Enfin, on peut noter que cet implant présente une conception qui est monobloc et qui ne présente pas de spécificité de mise en œuvre aussi compliquée et onéreuse que ce qu'un implant du type vis-cheville expansive peut présenter.

Les dépressions créent des espaces situés à l'intérieur du cône de révolution formé par la tête.

Ces espaces créés par les dépressions sont destinés à la fibro-intégration et permettent l'emprisonnement du cône en profondeur par les tissus mous colonisateurs. Les effets de la colonisation fibreuse sont ainsi optimisés par les moyens de blocage en rotation de l'implant autour de son axe d'insertion, selon l'invention.

La capacité de la tête à s'inscrire à l'intérieur d'un polygone, pouvant être un polygone régulier, permet d'optimiser les espaces destinés à la fibro-intégration. Les tissus colonisateurs permettent alors d'emprisonner le cône en profondeur et bloquent toute rotation du cône et ainsi de l'implant sous-talien autour de son axe d'insertion.

Les évidements augmentent la quantité d'espace situés à l'intérieur de la tête qui est dédiée à la fibro-intégration. Ces évidements augmentent aussi la capacité de l'implant à éviter toute rotation autour de son axe d'insertion.

Grâce à la création de la ou les cavités annulaires, le tissu mou colonisateur emprisonne la tête de l'implant. L'effet de blocage de la migration axiale est renforcé.

Cet effet de blocage est d'autant plus important que le fond de la ou les cavités annulaires permettent également d'inscrire un ou plusieurs polygones de fibro-intégration à l'intérieur du cône, ce qui bloque efficacement toute rotation, la ou les cavités annulaires prenant une forme polygonale.

En effet, on remarque alors une augmentation de la quantité de zone de butée destinées à coopérer avec le tissu colonisateur.

Avantageusement, les moyens de retenue en rotation comprennent au moins une arête s'étendant en longueur sur la surface radialement extérieure de la tête de manière essentiellement confondue ou parallèle à une génératrice de la surface radialement extérieure, créant une zone de butée destinée à coopérer avec le tissu colonisateur.

Grâce à la ou aux arêtes, suite à la colonisation par le tissu colonisateur de l'espace entourant l'implant, le tissu colonisateur exercera une résistance à l'encontre de tout dévissement éventuel de l'implant.

En effet, les arêtes s'étendant en longueur sur la surface radialement extérieure de la tête, de manière essentiellement confondue ou parallèle à une génératrice de la surface radialement extérieure, vont ainsi créer des zones de butée.

Ces zones de butée sont alors spécifiquement orientées de manière à lutter contre toute rotation de l'implant autour de son axe d'insertion.

Ainsi, les arêtes des moyens de retenue en rotation présentent une orientation telle que ces arêtes ne forment pas un pas de vis d'un filetage d'ancrage. Aussi, les arêtes des moyens de retenue en rotation, plutôt que de provoquer un dévissage de l'implant, lutteront contre un effet de dévissage de l'implant y compris en présence du filetage d'ancrage sur la surface de la tige.

En d'autres termes, les arêtes créent, au moins sur un côté desdites arêtes, une surface formant une zone de butée contre laquelle le tissu colonisateur viendra en contact et empêchera la rotation dans un sens de l'implant autour de son axe d'insertion.

Avantageusement, les moyens de retenue en rotation comprennent plusieurs arêtes décalées angulairement les unes par rapport.

Une pluralité d'arêtes permet d'amplifier l'effet de retenue de rotation de l'implant autour de l'axe d'insertion.

Les dépressions peuvent être plus ou moins prononcées.

Selon une première approche, les dépressions peuvent présenter un rayon de courbure plus important que celui de la surface radialement extérieure du cône de révolution.

Selon une deuxième approche, les dépressions peuvent correspondre à un méplat. Cette deuxième approche est bien entendu plus performante que la première.

Selon une troisième approche, les dépressions forment des concavités sur la surface radialement extérieure du cône de révolution. Cette troisième approche est également plus performante que la première approche.

Les concavités permettent d'augmenter l'espace dédié à la fibro-intégration, situé à l'intérieur de la forme théorique du cône de révolution.

Suivant une solution préférentielle, chaque dépression est délimitée par deux arêtes.

Les dépressions participent ainsi à la formation de ces arêtes. En effet, les bords des dépressions forment alors un plan dont l'intersection avec la surface du cône constituent les arêtes.

Les dépressions s'étendent alors sur toute la longueur des arêtes.

C'est-à-dire que les dépressions s'étendent elles aussi en longueur sur la surface radialement extérieure de la tête, de manière essentiellement confondue ou parallèle à une génératrice de la surface radialement extérieure.

De cette manière, la présence d'un volume important dédié à la fibro-intégration se couple à la création des zones de butée par les arêtes. En effet, une surface importante de la tête de l'implant forme une zone de butée, et cette zone de butée se voit alors mise en vis-à-vis d'un important volume dédié à la fibro-intégration, permettant ainsi la création d'une résistance importante pour lutter contre une rotation de l'implant autour de son axe d'insertion.

Préférentiellement, la tête présente sur sa surface radialement extérieure des portions lisses continues entre chaque extrémité de la tête, les portions lisses continues étant régulièrement réparties autour de la tête.

Dans ce cas, les dépressions sont situées entre deux portions lisses continues.

Ces portions lisses continues permettent d'assurer la pénétration homogène et progressive de la tête lors de l'implantation, et de limiter les irritations et l'érosion osseuse. Ces portions lisses continues forment une continuité du cône de révolution et permet ainsi d'avoir une surface lisse conservée entre les arêtes qui encadrent une dépression.

Dans une solution préférée, les moyens de retenue en rotation présentent de 3 à 10 dépressions, et plus préférentiellement 6 dépressions, selon une coupe transversale perpendiculaire à l'axe d'insertion.

Avantageusement, selon une coupe transversale perpendiculaire à l'axe d'insertion, les coins du polygone sont formés à proximité des arêtes.

Préférentiellement, le polygone est régulier.

Selon un mode de réalisation préférentiel de l'invention, chaque dépression présente une pluralité d'évidements.

Tel qu'expliqué précédemment, ces évidements augmentent la quantité d'espace situés à l'intérieur de la tête qui est dédiée à la fibro-intégration. Ces évidements augmentent aussi la capacité de l'implant à éviter toute rotation autour de son axe d'insertion.

Selon un mode de réalisation avantageux, l'implant comprend une succession de cavités annulaires situées le long de l'axe d'insertion à l'intérieur de la tête, les évidements situés à une même hauteur le long de l'axe d'insertion communiquant ensemble à l'intérieur de la tête pour créer l'une des cavités annulaires.

Ce mode de réalisation permet de maximiser l'ancrage de l'implant grâce à la formation de plusieurs polygones de fibro-intégration à l'intérieur du cône (les tissus colonisateurs à l'intérieur de chacune des cavités annulaires) communiquant avec l'extérieur de l'implant, tout en préservant de manière plus importante la surface extérieure de la tête de l'implant pour optimiser son contact avec les structures osseuses, et en étant facile à fabriquer.

Selon une solution préférée de l'invention, les dépressions :

- sont régulièrement réparties sur la surface radialement extérieure de la tête ;
- s'étendent en longueur de manière essentiellement parallèle à une génératrice de la surface radialement extérieure de la tête, sur au moins 70 % de sa longueur.

Avec des dépressions positionnées de telle manière et présentant une longueur d'au moins 70 % celle de la tête, les chances d'avoir un contact plan sur la surface de l'os sont augmentées. L'implant présente alors une meilleure stabilité une fois implanté.

De manière encore plus préférée, les dépressions s'étendent en longueur sur essentiellement toute la longueur de la tête.

Avantageusement, l'implant présente une canule centrale de guidage sur broche, la ou les cavités annulaires étant indépendantes de la canule centrale de guidage.

Grâce à une telle canule centrale de guidage sur broche, la mise en place de l'implant est aisée.

L'indépendance des cavités annulaires par rapport à la canule centrale permet d'optimiser l'ancrage de l'implant par le biais des polygones de fibro-intégration et des fonds des cavités annulaires accueillant les polygones de fibro-intégration.

Préférentiellement, l'implant présente également un taraudage à l'intérieur de la canule. Ce taraudage permet l'insertion d'une tige filetée destinée à se connecter à l'implant pour en faciliter le retrait.

Selon une caractéristique avantageuse :

- l'implant présente une longueur $L_{total}$ ;
- la tête présente une longueur $L_{tête}$,

avec $0,5 \leq \dfrac{L_{tête}}{L_{total}} \leq 0,8$, préférentiellement avec

$0,69 \leq \dfrac{L_{tête}}{L_{total}} \leq 0,77$.

D'autres caractéristiques et avantages de l'invention apparaîtront plus clairement à la lecture de la description suivante de différents modes de réalisation préférentiels de l'invention, donnés à titre d'exemples illustratifs et non limitatifs, et des dessins annexés parmi lesquels :

- la figure 1 est une représentation d'un implant sous-talien selon l'invention, inséré dans l'articulation talo-calcanéenne par son orifice latéral ;
- les figures 2a et 2b sont des représentations schématiques d'un premier mode de réalisation de l'implant, respectivement selon une vue latérale et une coupe longitudinale passant par l'axe d'insertion ;
- la figure 3 est une vue en perspective d'un deuxième mode de réalisation de l'invention ;
- la figure 4 est une représentation schématique d'un troisième mode de réalisation de l'invention, selon une vue latérale ;
- la figure 5 est une vue schématique de côté de l'implant selon un quatrième mode de réalisation ;
- la figure 6 est une vue schématique en perspective d'une partie de la tête de l'implant de la figure 5 ;
- la figure 7 est une vue en coupe de la figure 6.

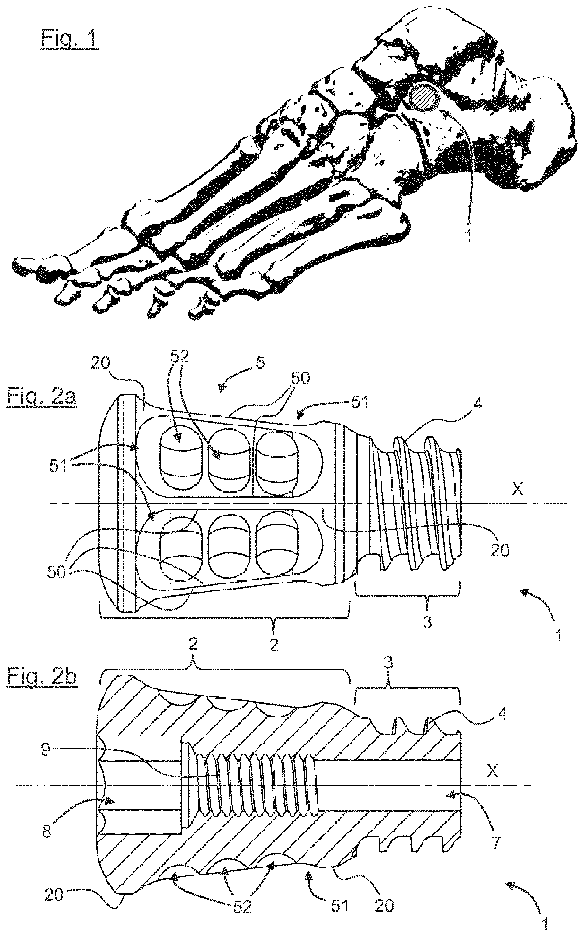

En référence aux figures 1 à 5, l'invention a pour objet un implant 1 sous-talien.

En référence à la figure 1, l'implant sous-talien selon l'invention est conçu pour l'arthrorise de l'articulation talo-calcanéenne. L'implant est destiné à être inséré dans l'articulation talo-calcanéenne par son orifice latéral.

En référence aux figures 2a, 2b, 3, 4 et 5, l'implant 1 sous-talien selon l'invention présente une forme oblongue qui s'étend longitudinalement selon un axe d'insertion X de l'implant dans l'articulation talo-calcanéenne.

Un tel implant est extra osseux.

Selon les figures 2a et 2b, 3, 4 et 5 l'implant 1 comporte :

- une tête 2 ;
- une tige 3.

En référence aux figures 2a, 2b, 3, 4 et 5, la tête 2 a une forme de cône de révolution centrée sur l'axe d'insertion X. Plus précisément, la tête 2 a une forme tronconique.

La tige 3, quant à elle, a une forme de cylindre de révolution centrée sur l'axe d'insertion.

La tête s'étend en s'évasant à partir de la tige en étant située en amont de la tête selon l'axe d'insertion X de l'implant.

En d'autres termes, selon l'axe d'insertion, la tige est située devant la tête, la tête présentant une forme de cône s'évasant à partir de la tige.

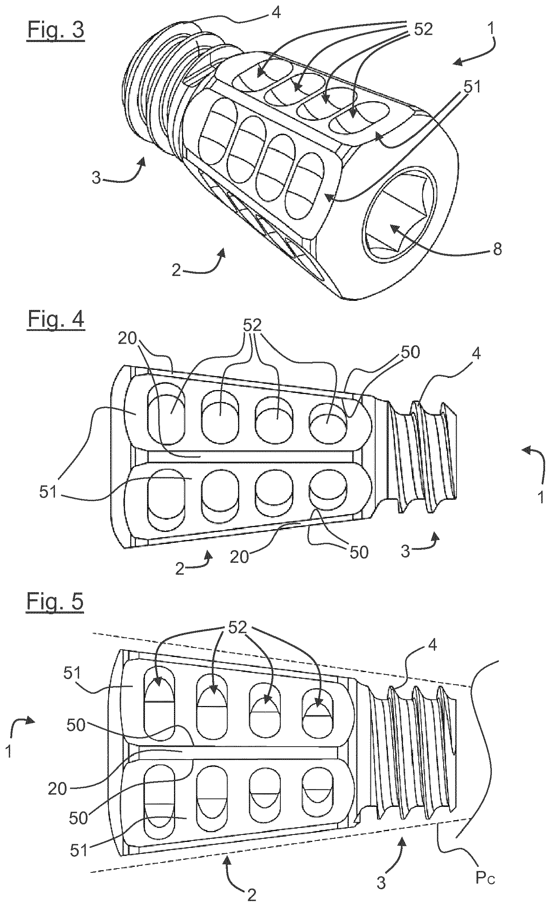

Tel qu'illustré par la figure 5 et tel qu'évoqué précédemment, la tête 2 a plus précisément une forme tronconique à partir de laquelle s'étend la tige 3. La tige 3 s'inscrit préférentiellement à l'intérieur de la projection $P_c$ de la forme de cône de révolution de la tête, à partir de la tête et en direction du sommet du cône.

Toujours selon les figures 2a à 5, l'implant 1 comporte :

- un filetage d'ancrage 4 ;
- des moyens de retenue en rotation 5 de l'implant autour de l'axe d'insertion.

Le filetage d'ancrage 4 est présenté par la tige à sa surface. La tige, avec à sa surface le filetage d'ancrage, est destinée à être insérée jusqu'à l'axe de rotation de l'articulation talo-calcanéenne.

La tête, quant à elle, présente à sa surface radialement extérieure les moyens de retenue en rotation 5.

Les moyens de retenue en rotation 5 sont destinés à exercer une retenue contre un tissu colonisateur.

En référence aux figures 2a, 4 et 6 et 7, les moyens de retenue en rotation 5 comprennent au moins une arête 50.

L'arête 50 s'étend en longueur sur la surface radialement extérieure 20 de la tête 2 de manière essentiellement confondue ou parallèle à une génératrice de la surface radialement extérieure de la tête. L'arête 50 crée une zone de butée destinée à coopérer avec le tissu colonisateur.

En effet, une arête est formée à l'intersection de deux plans. L'arête crée ainsi une zone de butée (ou « surface présentée par la surface de la tête »), qui ne correspond pas à la forme de cône de révolution dans laquelle ladite tête s'inscrit. Cette surface, suite à la colonisation des tissus mous, forme alors une butée coopérant avec le tissu colonisateur pour exercer une retenue en rotation de l'implant autour de son axe d'insertion.

L'arête, en s'étendant en longueur sur la surface radialement extérieure de la tête de manière essentiellement confondue ou parallèle à une génératrice de la surface radialement extérieure de la tête, ne peut pas être assimilée au filetage d'ancrage situé sur la tige.

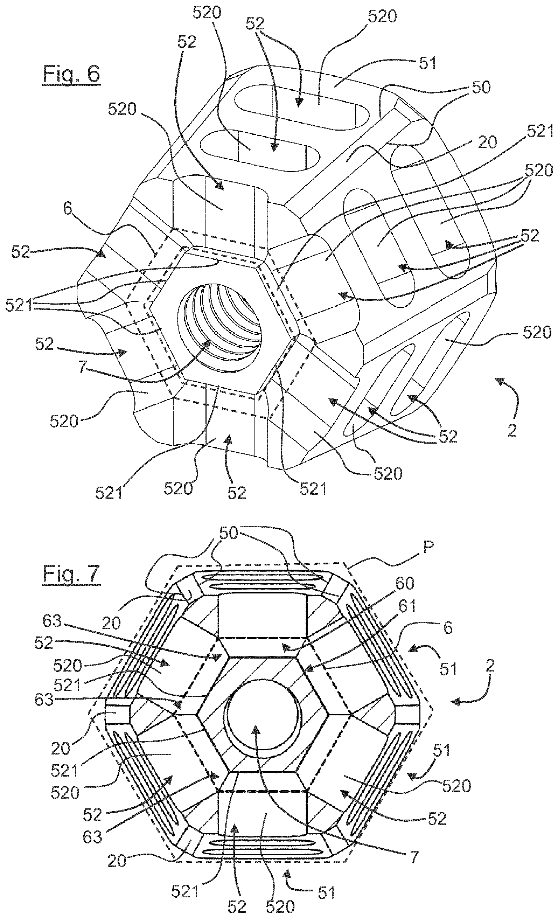

En référence à la figure 7, les moyens de retenue en rotation 5 comprennent plusieurs arêtes 50 décalées angulairement les unes par rapport aux autres.

Les moyens de retenue en rotation 5 comprennent également des dépressions 51 par rapport à la forme de cône de révolution de la tête. Ces dépressions correspondent plus précisément à un décroché de la surface extérieure de la tête par rapport à forme conique de révolution théorique dans laquelle s'inscrit la tête.

Selon le mode de réalisation illustré par les figures 2a et 2b, les dépressions 51 prennent la forme de rainures longitudinales qui sont essentiellement confondues ou parallèles à une génératrice de la surface radialement extérieure de la tête.

Selon le mode de réalisation illustré par la figure 3 ainsi que par les modes de réalisation illustrés par les figures 4, 5, 6 et 7, les dépressions 51 prennent la forme de méplats.

En référence aux figures 6 et 7, chaque dépression 51 (prenant la forme de méplats) est délimitée par deux arêtes 50.

En l'occurrence, lesdites arêtes sont formées lors de la création des méplats et correspondent à l'intersection des bords latéraux du méplat avec la surface du cône.

En référence aux figures 2a, 4, 5, 6 et 7, la tête 2 présente sur sa surface radialement extérieure des portions lisses continues 20 entre chaque extrémité de la tête. Ces portions lisses continues correspondent à la surface radialement extérieure 20 de la tête s'inscrivant dans le cône de révolution.

Ces portions lisses continues sont régulièrement réparties autour de la tête. Elles permettent ainsi de former une continuité du cône de révolution.

Tel qu'illustré par ces figures, chaque dépression est située entre deux portions lisses continues.

En référence à la figure 7, selon une coupe transversale perpendiculaire à l'axe d'insertion, les moyens de retenue en rotation présentent six dépressions.

Toujours selon la coupe transversale perpendiculaire à l'axe d'insertion, la tête s'inscrit à l'intérieur d'un polygone P dont les côtés s'étendent au niveau des dépressions et dont les coins sont formés à proximité des arêtes.

Selon le présent mode de réalisation le polygone P est régulier.

La capacité de la tête à s'inscrire dans un polygone permet son ancrage à l'intérieur d'un polygone de fibro-intégration.

Grâce à ce mode de réalisation, les tissus mous colonisateurs peuvent emprisonner le cône en profondeur et bloquer la rotation axiale.

Les dépressions peuvent aussi être mises en contact avec certaines surfaces osseuses lors de l'implantation. Une éventuelle rotation peut ainsi être bloquée de manière bien plus efficace.

En référence aux figures 2a à 7, les moyens de retenue en rotation 5 peuvent également comprendre des évidements 52 situés à l'intérieur des dépressions 51.

En référence aux figures 2a, 2b, 3 et 4, les évidements 52 sont borgnes.

En référence au mode de réalisation illustré par les figures 4, 5 et 6, les évidements 52 prennent une forme de rainure transversale perpendiculaire, ou essentiellement perpendiculaire à une génératrice de la surface radialement extérieure de la tête.

Selon les modes de réalisation illustrés par les figures 5 à 7, les évidements 52 communiquent ensemble à l'intérieur de la tête 2. Les évidements communiquent alors pour créer une ou plusieurs cavités annulaires 6 (illustrées par les figures 6 et 7) qui sont destinées à être colonisées par du tissu colonisateur.

Cette ou ces cavités annulaires prennent une forme de polygone, ou « forme polygonale ». La ou les cavités annulaires présentant plus précisément une forme hexagonale.

En effet, en référence aux figures 6 et 7, chaque évidement 52 est formé par une paroi périphérique 520 et par un fond 521, la paroi périphérique s'étendant à partir du fond 521 jusqu'à la surface de la tête 2 de l'implant 1. Le fond 521 de chaque évidement est ainsi formé en fonction de la profondeur de l'évidement 521. Ces fonds 521 sont plans.

Tel qu'illustré par ces figures, les fonds 521, aboutés les uns aux-autres présentent une forme de polygone (ou « section polygonale »), notamment une forme de polygone régulier, et encore plus précisément une forme hexagonale.

Les cavités annulaires 6 sont alors créées grâce à la formation de passages de communication 63 entre les évidements 52 qui sont adjacents les uns des autres. Ces passages de communication 63 sont en l'occurrence formés par la rencontre des parois périphériques 520.

En d'autre termes et en référence à la figure 7, les cavités annulaires 6 définissent un volume annulaire qui présente, selon une coupe transversale perpendiculaire à l'axe d'insertion, un périmètre interne 61 et un périmètre externe 60 (le périmètre interne 61 étant plus précisément formé par le fond 521 des évidements 52) qui ont tous deux une forme de polygone, notamment une forme de polygone régulier, et plus précisément une forme d'hexagone.

Selon le présent mode de réalisation illustré par les figures 6 et 7, les évidements situés à une même hauteur le long de l'axe d'insertion communiquent ensemble pour créer une cavité annulaire. La cavité annulaire créée est indépendante de celles créées par des évidements situés à une hauteur différente le long de l'axe d'insertion.

Selon un mode de réalisation non illustré, des évidements directement adjacents et situés à au moins deux hauteurs différentes peuvent créer une même cavité annulaire.

Tel qu'illustré par la figure 5, l'implant comprend une succession de cavités annulaires situées le long de l'axe d'insertion à l'intérieur de la tête 2.

Ces cavités annulaires 6 participent à la maximisation de l'espace situé à l'intérieur de la tête destiné à la fibro-intégration.

En référence aux figures 2b, 6 et 7, l'implant 1 présente une canule centrale de guidage 7 sur broche. Tel qu'illustré par les figures 6 et 7, la ou les cavités annulaires 6 sont indépendantes de la canule centrale de guidage.

Selon les figures 2b et 3, la tête 2 de l'implant 1 comprend également des moyens d'entraînement 8. Ces moyens d'entraînement 8 de l'implant prennent la forme d'une cavité arrière à six pans.

En référence à la figure 2b, les moyens d'entraînement 8 communiquent avec la canule centrale de guidage 7. Cette canule centrale de guidage 7 présente un taraudage 9 à l'intérieur de la canule. Ce taraudage permet le couplage de l'implant à une tige filetée destinée à permettre de faciliter le retrait de l'implant.

L'implant selon l'invention peut être réalisé en titane, en alliage de titane, en poly éthylène, en PEEK (polyétheréthercétone), ou encore en polymère bio résorbable (PLLA (acide (L)-lactique), PLA (acide polylactique), PGA (acide polyglycolique) ou hybride).

Tel qu'illustré par les figures, l'implant selon l'invention est monobloc. C'est-à-dire que l'implant est fabriqué d'une seule pièce.

Un tel implant selon l'invention peut être fabriqué par tournage/usinage. Il présente ainsi un coût de fabrication plus faible que des modes de fabrication par ajout de matière.

A titre indicatif :

- le diamètre à l'arrière de la tête peut être compris de 5 à 20 mm ;
- la longueur totale de l'implant est comprise de 10 à 20 mm ;
- la longueur de la tête est comprise de 5 à 16 mm.

De manière préférentielle, la longueur totale de l'implant est comprise de 13,5 à 18 mm et la longueur de la tête est comprise de 6,9 à 15,4 mm.

## Revendications

1. Implant (1) sous-talien pour arthrorise de l'articulation talo-calcanéenne, l'implant étant destiné à être inséré dans l'articulation talo-calcanéenne par son orifice latéral et présentant une forme oblongue qui s'étend longitudinalement selon un axe d'insertion (X) de l'implant, l'implant étant monobloc et comportant :

- une tête (2) avec une forme de cône de révolution centrée sur l'axe d'insertion ;
- un filetage d'ancrage (4) ; et
- une tige (3) avec une forme de cylindre de révolution centrée sur l'axe d'insertion, la tige présentant à sa surface le filetage d'ancrage et étant destinée à être insérée jusqu'à l'axe de rotation de l'articulation talo-calcanéenne, la tête s'étendant en s'évasant à partir de la tige et étant située en amont de la tête selon l'axe d'insertion de l'implant, la tête présentant à sa surface radialement extérieure des moyens de retenue (5) en rotation de l'implant autour de l'axe d'insertion, les moyens de retenue en rotation étant destinés à exercer une retenue contre un tissu colonisateur, et en ce que les moyens de retenue en rotation (5) comprennent :

- des dépressions (51) par rapport à la forme de cône de révolution de la tête (2) ;
- au moins un évidement (52) situé à l'intérieur des dépressions (51), le ou les évidements (52) communiquant ensemble à l'intérieur de la tête (2) pour créer au moins une cavité annulaire (6) destinée à être colonisée par du tissu colonisateur,

**caractérisé en ce que**, selon une coupe transversale perpendiculaire à l'axe d'insertion :

- la tête (2) s'inscrit à l'intérieur d'un polygone (P) dont les côtés s'étendent au niveau des dépressions (51) ;
- la ou les cavités annulaires présentent une forme polygonale apte à permettre l'inscription d'un polygone de fibro-intégration à l'intérieur de la tête.

2. Implant (1) selon la revendication précédente, **caractérisé en ce que** les moyens de retenue en rotation (5) comprennent au moins une arête (50) s'étendant en longueur sur la surface radialement extérieure de la tête (2) de manière essentiellement confondue ou parallèle à une génératrice de la surface radialement extérieure, créant une zone de butée destinée à coopérer avec le tissu colonisateur.

3. Implant (1) selon la revendication précédente, **caractérisé en ce que** les moyens de retenue en rotation (5) comprennent plusieurs arêtes (50) décalées angulairement les unes par rapport.

4. Implant (1) selon la revendication précédente, **caractérisé en ce que** chaque dépression (51) est délimitée par deux arêtes (50).

5. Implant (1) selon la revendication précédente, **caractérisé en ce que**, selon une coupe transversale perpendiculaire à l'axe d'insertion, les coins du polygone (P) sont formés à proximité des arêtes (50).

6. Implant (1) selon l'une quelconque des revendications précédentes, **caractérisé en ce que** le polygone (P) est régulier.

7. Implant (1) selon l'une quelconque des revendications précédentes, **caractérisé en ce que** chaque dépression (51) présente une pluralité d'évidements (52).

8. Implant (1) selon la revendication précédente, **caractérisé en ce qu'**il comprend une succession de cavités annulaires (6) situées le long de l'axe d'insertion (X) à l'intérieur de la tête (2), les évidements (52) situés à une même hauteur le long de l'axe d'insertion communiquant ensemble à l'intérieur de la tête (2) pour créer l'une des cavités annulaires (6).

9. Implant (1) selon l'une quelconque des revendications précédentes, **caractérisé en ce que** les dépressions (51) :

- sont régulièrement réparties sur la surface radialement extérieure de la tête (2) ;
- s'étendent en longueur de manière essentiellement parallèle à une génératrice de la surface radialement extérieure de la tête, sur au moins 70 % de sa longueur.

10. Implant (1) selon la revendication précédente, **caractérisé en ce que** les dépressions (51) s'étendent en longueur sur essentiellement toute la longueur de la tête (2).

11. Implant (1) selon la revendication précédente, **caractérisé en ce que** l'implant présente une canule centrale de guidage (7) sur broche, la ou les cavités annulaires (6) étant indépendantes de la canule centrale de guidage.

**Patentansprüche**

1. Subtalares Implantat (1) für Arthrorise des Sprunggelenks, wobei das Implantat ausgelegt ist, um in das Sprunggelenk durch seine seitliche Öffnung eingeführt zu werden, und eine längliche Form aufweist, die sich längs entlang einer Einführungsache (X) des Implantats erstreckt, wobei das Implantat aus einem Stück ist und Folgendes umfasst:

- einen Kopf (2) in Form eines Drehkegels, der auf der Einführungsachse zentriert ist;
- ein Verankerungsgewinde (4); und

- einen Schaft (3) in Form eines Drehzylinders, der auf der Einführungsachse zentriert ist, wobei der Schaft auf seiner Oberfläche das Verankerungsgewinde aufweist und ausgelegt ist, um bis zur Rotationsachse des Sprunggelenks eingeführt zu werden, wobei sich der Kopf ausgehend vom Schaft erstreckt und erweitert und sich vorgelagert vom Kopf gemäß der Einführungsachse des Implantats befindet, wobei der Kopf an seiner radial äußeren Oberfläche Rotationshaltmittel (5) des Implantats um die Einführungsachse aufweist, wobei die Rotationshaltmittel ausgelegt sind, um einen Halt gegen eine Besiedelungsgewebe auszuüben, und dadurch, dass die Rotationshaltmittel (5) Folgendes umfassen:

  - Vertiefungen (51) mit Bezug auf die Drehkegelform des Kopfs (2);
  - mindestens eine Aussparung (52), die sich im Inneren der Vertiefungen (51) befindet, wobei die Aussparung oder die Aussparungen (52) innerhalb des Kopfs (2) miteinander kommunizieren, um mindestens einen ringförmigen Hohlraum (6) zu erzeugen, der ausgelegt ist, um von Besiedelungsgewebe besiedelt zu werden,

  **dadurch gekennzeichnet, dass**, gemäß einem Querschnitt senkrecht zur Einführungsachse:

  - sich der Kopf (2) in das Innere eines Polygons (P) einschreibt, dessen Seiten sich auf der Ebene der Vertiefungen (51);
  - der oder die ringförmigen Hohlräume eine polygonale Form aufweisen, die ausgelegt ist, um die Einschreibung eines Polygons aus Faserintegration in das Innere des Kopfs zu ermöglichen.

2. Implantat (1) nach dem vorhergehenden Anspruch, **dadurch gekennzeichnet, dass** die Rotationshaltmittel (5) mindestens eine Kante (50) umfassen, der sich in der Länge auf der radial äußeren Oberfläche des Kopfs (2) auf im Wesentlichen überlagernde Weise oder parallel zu einem Generator auf der radial äußeren Oberfläche erstreckt, wodurch ein Anschlagbereich erzeugt wird, der ausgelegt ist, um mit dem Besiedlungsgewebe zusammenzuarbeiten.

3. Implantat (1) nach dem vorhergehenden Anspruch, **dadurch gekennzeichnet, dass** die Rotationshaltmittel (5) mehrere Kanten (50) umfassen, die winklig mit Bezug aufeinander versetzt sind.

4. Implantat (1) nach dem vorhergehenden Anspruch, **dadurch gekennzeichnet, dass** jede Vertiefung (51) von zwei Kanten (50) begrenzt ist.

5. Implantat (1) nach dem vorhergehenden Anspruch, **dadurch gekennzeichnet, dass** gemäß einem Querschnitt, der senkrecht zur Einführungsachse ist, die Ecken des Polygons (P) in der Nähe der Kanten (50) gebildet sind.

6. Implantat (1) nach einem der vorhergehenden Ansprüche, **dadurch gekennzeichnet, dass** das Polygon (P) regelmäßig ist.

7. Implantat (1) nach einem der vorhergehenden Ansprüche, **dadurch gekennzeichnet, dass** jede Vertiefung (51) eine Vielzahl von Aussparungen (52) aufweist.

8. Implantat (1) nach dem vorhergehenden Anspruch, **dadurch gekennzeichnet, dass** es eine Aufeinanderfolge von ringförmigen Hohlräumen (6) umfasst, die sich entlang der Einführungsachse (X) im Inneren des Kopfs (2) befinden, wobei die Aussparungen (52), die sich auf einer gleichen Höhe entlang der Einführungsache befinden, miteinander im Inneren des Kopfs (2) kommunizieren, um einen der ringförmigen Hohlräume (6) zu erzeugen.

9. Implantat (1) nach einem der vorhergehenden Ansprüche, **dadurch gekennzeichnet, dass** die Vertiefungen (51):

  - regelmäßig auf der radial äußeren Oberfläche des Kopfs (2) verteilt sind;
  - sich in der Länge auf im Wesentlichen parallele Weise zu einem Generator der radial äußeren Oberfläche des Kopfs auf mindestens 70 % seiner Länge erstrecken.

10. Implantat (1) nach dem vorhergehenden Anspruch, **dadurch gekennzeichnet, dass** die Vertiefungen (51) sich in der Länge auf im Wesentlichen der gesamten Länge des Kopfs (2) erstrecken.

11. Implantat (1) nach dem vorhergehenden Anspruch, **dadurch gekennzeichnet, dass** das Implantat eine zentrale Führungskanüle (7) auf Spindel umfasst, wobei der oder die ringförmigen Hohlräume (6) unabhängig von der zentralen Führungskanüle sind.

**Claims**

1. Subtalar Implant (1) for arthroereisis of the talocalcaneal joint, the implant being intended to be inserted into the talocalcaneal joint via its lateral orifice and having an oblong shape that extends longitudinally along an axis of insertion (X) of the implant, the implant being one-piece and comprising:

- a head (2) with the shape of a cone of revolution centred on the axis of insertion;
- an anchoring thread (4);

**characterised in that** it comprises a rod (3) having the shape of a cylinder of revolution centred on the axis of insertion, the rod having, on its surface, the anchoring thread and being intended to be inserted up to the axis of rotation of the talocalcaneal joint, the head flaring out from the rod and being located upstream of the head along the axis of insertion of the implant, the head having, on its radially outer surface, means (5) for retaining the implant in rotation about the axis of insertion, the means for retaining in rotation being intended to exert retention against a colonising tissue,
and **in that** the means (5) for retaining in rotation comprise:

- depressions (51) with respect to the shape of a cone of revolution of the head (2);
- at least one recess (52) located inside the depressions (51), the recess(es) (52) communicating with each other inside the head (2) in order to create at least one annular cavity (6) intended to be colonised by colonising tissue,

and **in that**, in a transverse cross-section perpendicular to the axis of insertion:

- the head (2) is inscribed inside a polygon (P), the sides of which extend at the depressions (51);
- the annular cavity or cavities have a polygonal shape suitable for allowing the inscription of a fibrous integration polygon inside the head.

2. Implant (1) according to the previous claim, **characterised in that** the means (5) for retaining in rotation comprise at least one edge (50) extending lengthwise on the radially outer surface of the head (2) in a manner substantially overlapping with or parallel to a generatrix of the radially outer surface, creating a stop zone intended to cooperate with the colonising tissue.

3. Implant (1) according to the previous claim, **characterised in that** the means (5) for retaining in rotation comprise a plurality of edges (50) angularly offset with respect to one another.

4. Implant (1) according to the previous claim, **characterised in that** each depression (51) is defined by two edges (50).

5. Implant (1) according to the previous claim, **charac-**

**terised in that** in a transverse cross-section perpendicular to the axis of insertion, the corners of the polygon (P) are formed near the edges (50).

6. Implant (1) according to any one of the previous claims, **characterised in that** the polygon (P) is regular.

7. Implant (1) according to any one of the previous claims, **characterised in that** each depression (51) has a plurality of recesses (52).

8. Implant (1) according to the previous claim, **characterised in that** it comprises a succession of annular cavities (6) located along the axis of insertion (X) inside the head (2), the recesses (52) located at the same height along the axis of insertion communicating with each other inside the head (2) in order to create one of the annular cavities (6).

9. Implant (1) according to any one of the previous claims, **characterised in that** the depressions (51):

- are regularly distributed over the radially outer surface of the head (2);
- extend lengthwise in a manner substantially parallel to a generatrix of the radially outer surface of the head, over at least 70% of its length.

10. Implant (1) according to the previous claim, **characterised in that** the depressions (51) extend lengthwise over substantially the entire length of the head (2).

11. Implant (1) according to the previous claim, **characterised in that** the implant has a central cannula (7) for guiding on a pin, the annular cavity or cavities (6) being independent of the central guide cannula.

Fig. 1

Fig. 2a

Fig. 2b

Fig. 3

Fig. 4

Fig. 5

Fig. 6

Fig. 7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8092547 B2
- US 20090099664 A1
- WO 2012100054 A1
- US 9125701 B2
- US 20130304224 A1